# EUROPÄISCHE PATENTSCHRIFT

(11) **EP 0 486 797 B1**
(45) Veröffentlichungstag und Bekanntmachung des Hinweises auf die Patenterteilung: **11.08.1993**
(21) Anmeldenummer: 91116921.7
(22) Anmeldetag: 04.10.1991
(51) Int. Cl.: C10G 7/08, C07C 7/08

(54) **Verfahren zur Aufarbeitung des Sumpfproduktes von Extraktivdestillationsprozessen zur Gewinnung reiner Aromaten**
Process for upgrading the residues from extractive destillation processes for the recovery of pure aromatics
Procédé pour la révalorisation des résidus de procédés de destillation extractive pour l'obtention d'hydrocarbures aromatiques

(30) Priorität: 22.11.1990 DE 4037060
(43) Veröffentlichungstag der Anmeldung: 27.05.1992
(73) Patentinhaber: Krupp Koppers GmbH, 45143 Essen (DE)
(72) Erfinder: Skatulla, Luzian, W-4330 Mülheim/Ruhr (DE); Schneider, Hans-Christoph, W-4320 Hattingen (DE); Vollmer, Hans-Jürgen, Dr., W-4300 Essen 1 (DE)

(56) Entgegenhaltungen:
- EP-A- 0 046 876

## Beschreibung

Die Erfindung betrifft ein Verfahren zur Aufarbeitung des Sumpfproduktes von Extraktivdestilllationsprozessen zur Gewinnung reiner Aromaten, bei dem das aufzuarbeitende Sumpfprodukt in eine mit Böden sowie Sumpf- und Seitenkocher versehene Abtriebskolonne eingeleitet wird, in der die zu gewinnenden Aromaten über Kopf abdestilliert werden, während das Lösungsmittel aus dem Sumpf der Abtriebskolonne abgezogen und nach indirektem Wärmeaustausch mit anderen Produktströmen zur Lösungsmittelaufgabe der Extraktivdestillationskolonne zurückgeführt wird.

Die Extraktivdestillation ist heute ein in der Praxis weit verbreitetes Verfahren zur Gewinnung von reinen Aromaten aus diese Verbindungen enthaltenden Einsatzkohlenwasserstoffgemischen. Für die Durchführung derartiger Extraktivdestillationsverfahren sind bereits eine Vielzahl unterschiedlicher selektiver Lösungsmittel vorgeschlagen worden, wobei sich N-substituierte Morpholine, insbesondere das N-Formylmorpholin, besonders bewährt haben. Die Trennwirkung des verwendeten selektiven Lösungsmittels beruht dabei darauf, daß durch seine Gegenwart die Dampfdrücke der einzelnen Komponenten des zu trennenden Stoffgemisches in der Weise verändert werden, daß die Dampfdruckunterschiede zwischen den Komponenten, die sich im Extrakt anreichern sollen, und jenen Komponenten, die sich im Raffinat anreichern sollen, vergrößert werden. Dadurch können die als leichter siedende Fraktion anfallenden Nichtaromaten über Kopf aus der Extraktivdestillationskolonne abdestilliert werden, während die Aromaten als schwerer siedende Komponenten zusammen mit dem Lösungsmittel als Sumpfprodukt in der Extraktivdestillationskolonne (sogenannte Extraktphase) anfallen. Um die zu gewinnenden Aromaten vom Lösungsmittel abzutrennen,ist deshalb die eingangs beschriebene Aufarbeitung des Sumpfproduktes der Extraktivdestillationskolonne in einer nachgeschalteteten Abtriebskolonne erforderlich.

Der Abtrieb der Aromaten aus dem Sumpfprodukt der Extraktivdestillationskolonne ist dabei stets mit einem großen Wärmeeinsatz verbunden, wobei die Wärme hauptsächlich zur Abtreibung der Aromaten aus dem Lösungsmittel im Unterteil der Abtriebskolonne sowie zur destillativen Abtrennung der Aromaten von Lösungsmittelresten im Oberteil der Abtriebskolonne benötigt wird. Da jedoch der Wärmebedarf beim Betrieb der Abtriebskolonne einen erheblichen Anteil an den Betriebsmittelkosten des Gesamtverfahrens ausmacht, sollte für den Betrieb der Abtriebskolonne eine Methode gefunden werden, die eine Aufarbeitung des Sumpfproduktes aus der Extraktivdestillationskolonne mit geringerem Wärmebedarf und damit niedrigeren Energiekosten ermöglicht. Hierbei soll insbesondere eine Arbeitsweise der Abtriebskolonne angestrebt werden, die sich durch eine hohe Ausnutzung der eingesetzten Wärme bei möglichst geringem apparativen Aufwand auszeichnet.

Bei der Durchführung von Extraktivdestillationsprozessen zur Gewinnung reiner Aromaten ist es zwar bereits bekannt, den Wärmeinhalt des aus dem Sumpf der Abtriebskolonne abfließenden heißen Lösungsmittels in der Weise auszunutzen, daß man das Lösungsmittel vor seiner Wiedereinleitung in die Extraktivdestillationskolonne im indirekten Wärmeaustausch mit anderen Produktströmen kühlt. So wird beispielsweise in einer von der Anmelderin herausgegebenen Werbeschrift (Koppers Bericht 333 b v. IX. 69) ein Verfahrensschema zur Gewinnung von reinem o-Xylol aus Reformaten durch Extaktivdestillation beschrieben, bei dem das aus der Abtriebskolonne ablaufende Lösungsmittel zunächst im indirekten Wärmeaustausch zur Aufheizung des Sumpfproduktes der Abtriebskolonne und der Extraktivdestillationskolonne mit herangezogen wird. Durch eine derartige Arbeitsweise wird jedoch das Lösungsmittel nicht in dem Umfange abgekühlt, daß seine Wiedereinleitung in die Extraktivdestillationskolonne ohne weiteres möglich ist. Es muß deshalb vor der Wiedereinleitung in die Extraktivdestillationskolonne zunächst noch eine weitere Abkühlung in einem Luftkühler erfahren, wodurch natürlich ein Teil seines Wärmeinhaltes ungenützt verlorengeht. Ein indirekter Wärmeaustausch zwischen dem aus der Extraktivdestillationskolonne abgezogenen Sumpfprodukt und anderen Produktströmen ist bei diesem Verfahrensschema ebenfalls nicht vorgesehen.

Das der Lösung der weiter oben beschriebenen Aufgabe dienende Verfahren der eingangs genannten Art ist deshalb gekennzeichnet durch die Anwendung der Merkmale a) bis e) des Hauptanspruches.

Die Erfindung geht dabei von der Erkenntnis aus, daß bisher der hohe Wärmebedarf beim Betrieb der Abtriebskolonne vor allem dadurch verursacht wurde, daß das Sumpfprodukt aus der Extraktivdestillationskolonne mit zu hoher Temperatur in die Abtriebskolonne eingeleitet wird. Dadurch verdampft bei der Entspannung des Sumpfproduktes im Oberteil der Abtriebskolonne eine erhebliche Menge des Lösungsmittels, so daß ein hoher Rückfluß erforderlich ist, um bei vorgegebener Bodenzahl ein völlig lösungsmittelfreies Aromatendestillat zu erhalten. Andererseits ist der Wärmebedarf, der zum Abtrieb der Aromaten aus dem Lösungsmittel im Unterteil der Abtriebskolonne benötigt wird, umso geringer, je höher die Temperatur des dem Unterteil der Abtriebskolonne zufließenden Produktstromes ist. Durch die Anwendung des erfindungsgemäßen Verfahrens gelingt es, diesem Sachverhalt in der Weise Rechnung zu tragen, daß das Sumpfprodukt aus der Extraktivdestillationskolonne mit verhältnismäßig niedriger Temperatur in den Oberteil der Abtriebskolonne eingeleitet und die beim Abkühlen des eingeleiteten Sumpfproduktes frei werdende Wärme zur Beheizung des vom Oberteil in den Unterteil der Abtriebskolonne abfließenden Rückflusses verwendet wird.

Wie weit die Abkühlung des Sumpfproduktes aus der Extraktivdestillationskolonne dabei erfolgt, hängt natürlich in erster Linie von der Zusammensetzung des Sumpfproduktes und den Betriebsbedingungen der Abtriebskolonne ab. Es ist jedoch davon auszugehen, daß die Eintrittstemperatur des Sumpfproduktes in die Abtriebskolonne in jedem Falle zwischen 105 und 120°C liegen soll. Bei Anwendung von N-Formylmorpholin als selektivem Lösungsmittel liegt diese Eintrittstemperatur vorzugsweise zwischen 110 und 115°C.

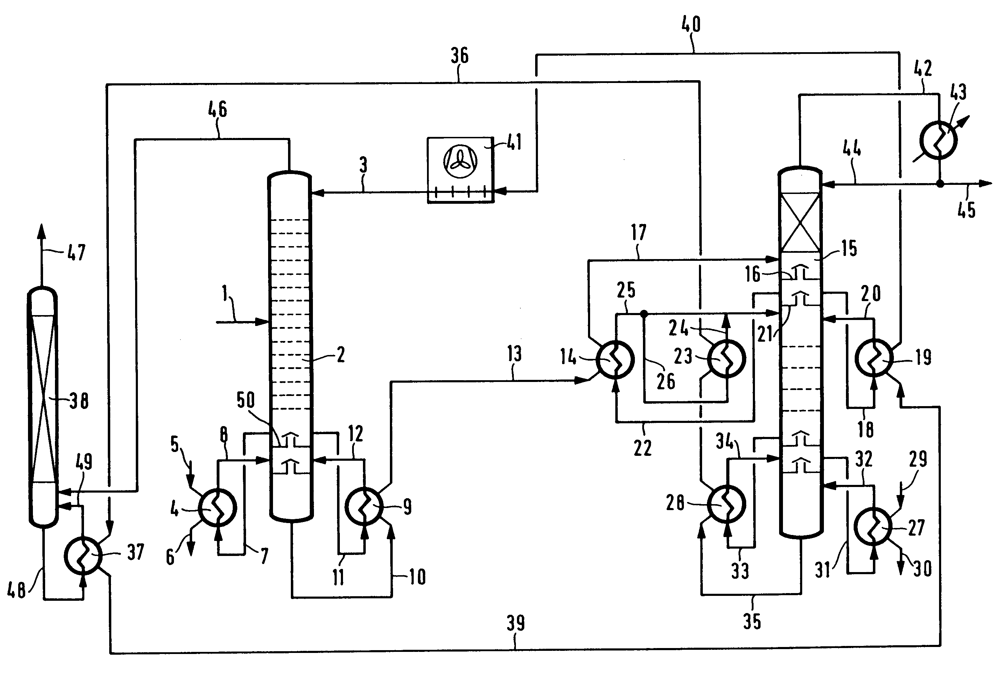

Weitere Einzelheiten des erfindungsgemäßen Verfahrens ergeben sich aus den vorliegenden Unteransprüchen und sollen nachfolgend anhand des in der Abbildung dargestellten Fließschemas erläutert werden. In diesem Fließschema sind selbstverständlich nur die zur Erläuterung des Verfahrensablaufes unerläßlichen Anlagenteile dargestellt, während sonstige Nebeneinrichtungen, wie Pumpen, Ventile sowie Meß- und Regeleinrichtungen, nicht aufgeführt werden.

Das aufzuarbeitende Einsatzprodukt, aus dem die Aromaten gewonnen werden sollen, wird hierbei über die Leitung 1 in den mittleren Teil der mit Böden versehenen Extraktivdestillationskolonne 2 eingeleitet. Das selektive Lösungsmittel wird über die Leitung 3 auf den Kopf der Extraktivdestillationskolonne 2 aufgegeben. Für die Beheizung der Extraktivdestillationskolonne 2 ist am Kolonnensumpf der Sumpfumlaufkocher 4 vorgesehen, in dem eine indirekte Beheizung des Sumpfproduktes mit Mitteldruckdampf erfolgt, der über die Leitung 5 zu- und über die Leitung 6 abgeführt wird. Das aufzuheizende Sumpfprodukt wird über die Leitung 7 in den Sumpfumlaufkocher 4 eingeleitet und über die Leitung 8 aus diesem abgezogen und in die Extraktivdestillationskolonne 2 zurückgeführt. Zusätzlich ist im unteren Bereich der Extraktivdestillationskolonne 2 der erste Seitenkocher 9 angeordnet, in den erfindungsgemäß das über die Leitung 10 aus der Extraktivdestillationskolonne 2 abgezogene Sumpfprodukt eingeleitet wird. Hier wird das Sumpfprodukt im indirekten Wärmeaustausch mit dem flüssigen Produktstrom gekühlt, der auf dem Boden 50 im unteren Bereich der Extraktivdestillationskolonne 2 anfällt. Dieser Produktstrom wird dabei vollständig über die Leitung 11 abgezogen und nach Passieren des ersten Seitenkochers 9 über die Leitung 12 unterhalb des Bodens 50 in die Extraktivdestillationskolonne 2 zurückgeführt. Das entsprechend vorgekühlte Sumpfprodukt gelangt anschließend über die Leitung 13 in den zweiten Seitenkocher 14, in dem es eine weitere Abkühlung im indirekten Wärmeaustausch mit der vom Kaminboden 21 aus der Abtriebskolonne 15 ablaufenden flüssigen Phase erfährt. Nach Passieren des Seitenkochers 14 liegt die Temperatur des Sumpfproduktes innerhalb des weiter oben genannten Temperaturbereiches, so daß das Sumpfprodukt mit verhältnismäßig niedriger Temperatur über die Leitung 17 in die Abtriebskolonne 15 eingeleitet werden kann. Der Aufgabeboden 16 für das Sumpfprodukt ist dabei erfindungsgemäß als Kaminboden ausgebildet und im oberen Teil der Abtriebskolonne 15, etwa 20 Böden unterhalb des Kolonnenkopfes, angeordnet. Die mit dem Einleiten des Sumpfproduktes in die Abtriebskolonne 15 verbundene Entspannung bewirkt eine weitere Temperaturabsenkung sowie die teilweise Verdampfung des eingeleiteten Sumpfproduktes. Infolge der erfindungsgemäßen Abkühlung des Sumpfproduktes verringert sich der Lösungsmittelanteil in der erzeugten Dampfphase, so daß der erforderliche Rückfluß vom Kopf der Abtriebskolonne 15 bei unveränderter Bodenzahl entsprechend gesenkt werden kann. Dieser Rückfluß wird zusammen mit der flüssigen Phase des eingeleiteten Sumpfproduktes auf dem Aufgabeboden 16 aufgefangen und über die Leitung 18 in den Seitenkocher 19 abgeleitet. In diesem Seitenkocher 19 wird diesem Produktstrom die für die Verdampfung des Rückflusses erforderliche Wärme im indirekten Wärmeaustausch mit heißem Lösungsmittel zugeführt. Anschließend wird der aufgeheizte Produktstrom über die Leitung 20 in die Abtriebskolonne 15 unterhalb des Aufgabebodens 16 zurückgeführt. Die sich hierbei auf dem unterhalb des Aufgabebodens 16 angeordneten Kaminboden 21 sammelnde flüssige Phase wird über die Leitung 22 aus der Abtriebskolonne 15 abgezogen und durch den Seitenkocher 14 geleitet. In diesem wird der flüssige Produktstrom teilweise verdampft und anschließend über die Leitung 25 unterhalb des Kaminbodens 21 in den Unterteil der Abtriebskolonne 15 eingeleitet. Gemäß einer besonderen Ausführungsform der Erfindung kann neben dem Seitenkocher 14, der wie bereits beschrieben mit dem vom Seitenkocher 9 kommenden Sumpfprodukt beheizt wird, noch ein weiterer Seitenkocher 23 vorhanden sein, der mit dem aus dem Sumpf der Abtriebskolonne 15 abgezogenen heißen Lösungsmittel beheizt wird. In diesem Falle gelangt der flüssige Produktstrom nach Passieren des Seitenkochers 14 über die Leitungen 25 und 26 in den Seitenkocher 23 und anschließend über die Leitung 24 zurück zur Leitung 25.

Der über die Leitung 25 in den Unterteil der Abtriebskolonne 15 wieder eingeleitete Produktstrom dient als Rückfluß für die aus dem Kolonnensumpf aufsteigenden Dämpfe. Zur Kolonnenbeheizung sind der Sumpfumlaufkocher 27 sowie ein zusätzlicher Seitenkocher 28 vorgesehen, wobei der Sumpfumlaufkocher 27 mit Hochdruckdampf und der Seitenkocher 28 mit heißem Lösungsmittel beaufschlagt wird. Die Leitungen 29 und 30 dienen der Zu- und Abführung von Hochdruckdampf zum Sumpfumlaufkocher 27, während die Leitungen 31 und 32 bzw 33 und 34 der Zu- und Abführung der Kolonnensumpfströme zu den entsprechenden Kochern dienen.

Das von den Aromaten befreite Lösungsmittel wird aus dem Sumpf der Abtriebskolonne 15 über die Leitung 35 abgezogen und gelangt anschließend nach Passieren der Seitenkocher 28 und 23 über die Leitung 36 in den Kocher 37 der sogenannten Raffinatdestillationskolonne 38. Von dort fließt das Lösungsmittel über die Leitung 39 zum Seitenkocher 19. Nach Passieren desselben gelangt es über die Leitung 40 zum Luftkühler 41, in dem es nur noch von Fall zu Fall einer geringfügigen Abkühlung unterworfen werden muß, bevor es über die Leitung 3 in die Extraktivdestillationskolonne 2 wieder eingeleitet werden kann. Das heißt beim erfindungsgemäßem Verfahren ist an insgesamt vier verschiedenen Stellen eine Wärmerückgewinnung aus dem Lösungsmittel vorgesehen, so daß dessen Wärmeinhalt in optimaler Weise genutzt werden kann.

Die vom Lösungsmittel befreiten Aromaten werden über Kopf durch die Leitung 42 aus der Abtriebskolonne 15 abgezogen und im Kühler 43 kondensiert. Anschließend wird ein Teilstrom über die Leitung 44 als Rückfluß auf die Abtriebskolonne 15 wieder aufgegeben, während der übewiegende Teil der Aromaten über die Leitung 45 aus dem Verfahren abgezogen und seiner weiteren Verwendung bzw. Verarbeitung zugeführt wird.

Die über Kopf aus der Extraktivdestillationskolonne 2 abgezogenen Nichtaromaten gelangen über die Leitung 46 in die sogenannte Raffinatdestillationskolonne 38, in der sie destillativ von den noch vorhandenen Lösungsmittelresten abgetrennt werden. Die lösungsmittelfreien Nichtaromaten werden als Kopfprodukt über die Leitung 47 aus der Raffinatdestillationskolonne 38 abgezogen. Am Fuß dieser Kolonne ist der Kocher 37 angeordnet, der, wie bereits gesagt wurde, im indirekten Wärmeaustausch mit dem über die Leitung 36 zugeführten heißen Lösungsmittel beheizt wird. Der Kocher 37 ist über die Leitung 48 für die Zufuhr und über die Leitung 49 für die Abfuhr mit der Raffinatdestillationskolonne 38 verbunden. Der aus dem Sumpf dieser Kolonne abgezogene lösungsmittelreiche Produktstrom kann entweder direkt in die Extraktivdestillationskolonne 2 zurückgeführt werden, oder er wird in den Lösungsmittelkreislauf in Leitung 39 eingespeist. Beide Möglichkeiten sind in der Abbildung nicht dargestellt.

Bei der Durchführung des erfindungsgemäßten Verfahrens wird durch das Zusammenwirken der verschiedenen Wärmeaustauschprozesse eine nicht unerhebliche Energieeinsparung erzielt. So beträgt beispielsweise bei einer Aromatengewinnungsanlage mit einer Durchsatzkapazität von 14,5 t/h die Einsparung an Fremdwärme (Hochdruckdampf) im Sumpfumlaufkocher 27 der Abtriebskolonne 1128 Gcal/h, was einer Einsparung von ca. 65 % entspricht. Zwar wird ein Teil dieser Energieeinsparung dadurch kompensiert, daß bei Anwendung des erfindungsgemäßen Verfahrens bei der Beheizung des Sumpfumlaufkochers 4 der Extraktivdestillationskolonne ein etwas höherer Bedarf an Mitteldruckdampf anftritt, da das aus dem Sumpf der Abtriebskolonne abgezogene heiße Lösungsmittel in diesem Falle nicht durch den Seitenkocher der Extraktivdestillationskolonne, sondern durch die an der Abtriebskolonne angeordneten Seitenkocher 28, 23 und 19 geleitet wird. Trotzdem verbleibt in diesem Falle insgesamt gesehen eine Energieeinsparung von ca. 21 %. Da diese Verringerung des Energiebedarfs ebenfalls eine Einsparung beim Kühlwasserverbrauch in derselben Größenordnung bewirkt, wird durch die Anwendung des erfindungsgemäßen Verfahrens eine nicht unerhebliche Verbesserung der Wirtschaftlichkeit des Gesamtverfahrens erreicht.

## Patentansprüche

1. Verfahren zur Aufarbeitung des Sumpfproduktes von Extraktivdestillationsprozessen zur Gewinnung reiner Aromaten, bei dem das aufzuarbeitende Sumpfprodukt in eine mit Böden sowie Sumpf- und Seitenkocher versehene Abtriebskolonne eingeleitet wird, in der die zu gewinnenden Aromaten über Kopf abdestilliert werden, während das Lösungsmittel aus dem Sumpf der Abtriebskolonne abgezogen und nach indirektem Wärmeaustausch mit anderen Produktströmen zur Lösungsmittelaufgabe der Extraktivdestillationskolonne zurückgeführt wird, gekennzeichnet durch folgende Merkmale:
a) Das aus der Extraktivdestillationskolonne abgezogene Sumpfprodukt wird vor seiner Einleitung in die Abtriebskolonne durch zwei hintereinander geschaltete Seitenkocher geleitet, in denen es im indirekten Wärmetausch mit aus dem Verfahren stammenden Produktströmen bis auf eine Temperatur zwischen 105 und 120°C gekühlt wird;
b) der auf einem Boden im unteren Bereich der Extraktivdestillationskolonne anfallende flüssige Produktstrom wird durch den ersten der Abkühlung des Sumpfproduktes dienenden Seitenkocher geleitet und anschließend in die Extraktivdestillationskolonne zurückgeführt;
c) die Einleitung des entsprechend abgekühlten Sumpfproduktes erfolgt im oberen Teil der Abtriebskolonne auf einen als Kaminboden ausgebildeten Aufgabeboden;
d) der Rückfluß aus dem Oberteil der Abtriebskolonne wird auf dem Aufgabeboden aufgefangen und in einen Seitenkocher abgeleitet, in dem er im indirekten Wärmeaustausch mit heißem Lösungsmittel aus dem Sumpf der Abtriebskolonne erwärmt und anschließend auf den Boden unterhalb des Aufgabebodens in die Abtriebskolonne wiedereingeleitet wird, wobei die auf diesem Boden anfallende flüssige Phase durch den der Abkühlung des Sumpfproduktes aus der Extraktivdestillationskolonne dienenden zweiten Seitenkocher geleitet und teilverdampft wird;
und
e) das bei der Teilverdampfung in der vorhergehenden Verfahrensstufe anfallende Dampf-Flüssigkeitsgemisch wird in den unteren Teil der Abtriebskolonne eingeleitet, wobei die Dämpfe in den oberen Teil der Abtriebskolonne gelangen, während die von den Dämpfen abgetrennte Flüssigkeit als Rückfluß im unteren Teil der Abtriebskolonne dient.

2. Verfahren nach Anspruch 1, **dadurch gekennzeichnet**, daß das Sumpfprodukt aus der Extraktivdestillationskolonne vorzugsweise mit einer Eintrittstemperatur zwischen 110 und 115°C in die Abtriebskolonne eingeleitet wird.

3. Verfahren nach den Ansprüchen 1 und 2, **dadurch gekennzeichnet**, daß in der Verfahrensstufe d) der flüssige Produktstrom aus der Abtriebskolonne nach Passieren des der Abkühlung des Sumpfproduktes aus der Extraktivdestillationskolonne dienenden zweiten Seitenkochers zusätzlich durch einen mit heißem Lösungsmittel aus dem Sumpf der Abtriebskolonne beaufschlagten Seitenkocher geleitet wird.

4. Verfahren nach den Ansprüchen 1 bis 3, **dadurch gekennzeichnet**, daß das aus dem Sumpf der Abtriebskolonne abgezogene heiße Lösungsmittel zusätzlich durch einen am Unterteil der Abtriebskolonne angeordneten Seitenkocher geleitet und im indirekten Wärmeaustausch mit einem Produktstrom aus dem Sumpf der Abtriebskolonne gekühlt wird

## Claims

1. Method of working-up the bottom product of extractive distillation processes to recover pure aromatics, wherein the bottom product to be worked up is introduced into a stripping column provided with trays and with a bottom and side boiler, in which stripping column the aromatics to be recovered are distilled off at the top, while the solvent is withdrawn from the bottom of the stripping column and, after indirect heat exchange with other product streams, is recycled into the extractive distillation column as solvent feed, said method being characterised by the following features:
a) Before it is introduced into the stripping column, the bottom product withdrawn from the extractive distillation column is passed through two side boilers connected in series, where it is cooled to a temperature of between 105 and 120°C by indirect heat exchange with product streams originating from the process;
b) the liquid product stream obtained on a tray in the lower region of the extractive distillation column is passed through the first side boiler serving to cool the bottom product, and is then recycled into the extractive distillation column;
c) the now cooled bottom product is introduced into the upper section of the stripping column on to a feed tray in the form of a riser tray;
d) the reflux from the upper section of the stripping column is collected on the feed tray and led into a side boiler, where it is heated by indirect heat exchange with hot solvent from the bottom of the stripping column, and is then reintroduced into the stripping column on to the tray below the feed tray, the liquid phase obtained on this tray being passed through the second side boiler serving to cool the bottom product from the extractive distillation column, and being partially evaporated; and
e) the vapour-liquid mixture obtained from the partial evaporation in the previous process step is introduced into the lower section of the stripping column, the vapours passing into the upper section of the stripping column, while the liquid separated from the vapours serves as reflux in the lower section of the stripping column.

2. Method according to Claim 1, **characterised in that** the bottom product from the extractive distillation column is preferably introduced into the stripping column at an inlet temperature of between 110 and 115°C.

3. Method according to Claims 1 and 2, **characterised in that,** in process step d), after passage through the second side boiler serving to cool the bottom product from the extractive distillation column, the liquid product stream from the stripping column is additionally passed through a side boiler supplied with hot solvent from the bottom of the stripping column.

4. Method according to Claims 1 to 3, **characterised in that** the hot solvent withdrawn from the bottom of the stripping column is additionally passed through a side boiler located in the lower section of the stripping column, and is cooled by indirect heat exchange with a product stream from the bottom of the stripping column.

## Revendications

1. Procédé pour retraiter le produit de queue du processus de distillation extractive en vue de l'obtention de fractions aromatiques pures, dans lequel le produit de queue à traiter est introduit dans la colonne de strippage qui est munie de plateaux, ainsi que de bouilleurs latéraux et d'un bouilleur à sa base, et dans laquelle les fractions aromatiques à obtenir sont séparées par distillation à la tête de ladite colonne, tandis que le solvant est soutiré du pied de la colonne de strippage et recyclé, pour l'alimentation en solvant de la colonne de distillation extractive, après un échange indirect de la chaleur avec d'autres flux de produit, ledit procédé se distinguant par les traits caractéristiques suivants :
a) Le produit soutiré au pied de la colonne de distillation extractive passe, avant d'être introduit dans la colonne de strippage, à travers deux bouilleurs latéraux montés en série l'un derrière l'autre, dans lesquels il est refroidi jusqu'à une température comprise entre 105 et 120°C par un échange indirect de chaleur avec des flux de produit provenant du procédé ;
b) le flux de produit s'accumulation sur un plateau dans la partie inférieure de la colonne de distillation extractive passe à travers le premier bouilleur latéral servant à refroidir le produit de queue, puis il est renvoyé dans la colonne de distillation extractive ;
c) l'introduction du produit de queue refroidi en conséquence intervient dans la partie supérieure de la colonne de strippage sur un plateau de chargement dont la forme est celle d'un plateau à cheminée ;
d) le reflux s'écoulant de la partie supérieure de la colonne de strippage est recueilli sur le plateau de chargement et évacué dans un bouilleur latéral dans lequel il est échauffé, par échange indirect de chaleur, avec du solvant très chaud provenant du pied de la colonne de strippage, puis il est réintroduit dans la colonne de strippage sur le plateau situé au-dessous du plateau de chargement, tandis que la phase liquide recueilie sur ce dernier plateau est conduite dans le second bouilleur latéral servant à refroidir le produit de queue de la colonne de distillation extractive, dans lequel elle s'évapore partiellement ;
et
e) le mélange de vapeur et de liquide se formant lors de l'évaporation partielle dans la phase précédente du procédé est introduit à la base de la colonne de strippage, étant entendu que les vapeurs parviennent dans la partie supérieure de la colonne de strippage, alors que le liquide séparé des vapeurs sert de reflux dans la partie inférieure de la colonne de strippage.

2. Procédé suivant la revendication 1, caractérisé par le fait que le produit de queue de la colonne de distillation extractive est introduit dans la colonne de strippage de préférence à une température d'admission comprise entre 110 et 115°C.

3. Procédé suivant les revendications 1 et 2, caractérisé par le fait qu'à la phase d) du procédé, le flux de produit liquide provenant de la colonne de strippage passe complémentairement, après avoir traversé le second bouilleur latéral servant à refroidir le produit soutiré au pied de la colonne de distillation extractive, par un bouilleur latéral alimenté par du solvant très chaud provenant du pied de la colonne de strippage.

4. Procédé suivant les revendications 1 à 3, caractérisé par le fait que le solvant très chaud soutiré du pied de la colonne de strippage passe complémentairement à travers un bouilleur latéral agencé à la partie inférieure de la colonne de strippage et y est refroidi par échange indirect de chaleur avec un flux de produit provenant du pied de la colonne de strippage.
